(19) Europäisches Patentamt
European Patent Office
Office européen des brevets

(11) Numéro de publication: 0 185 066 B1

# (12) FASCICULE DE BREVET EUROPEEN

(45) Date de publication du fascicule du brevet: **20.04.88**

(51) Int. Cl.[4]: **C 07 C 149/243**

(21) Numéro de dépôt: **85903007.4**

(22) Date de dépôt: **21.06.85**

(86) Numéro de dépôt international: **PCT/FR 85/00164**

(87) Numéro de publication internationale: **WO 86/00304 (16.01.86** Gazette **86/2)**

(54) **DERIVES SOUFRES DE L'ACIDE PARA-METHOXYCINNAMIQUE, PROCEDES D'OBTENTION, COMPOSITIONS DERMO-PHARMACEUTIQUES ET COSMETIQUES LES CONTENANT ET APPLICATIONS.**

(30) Priorité: **22.06.84 FR 8410009**

(43) Date de publication de la demande: **25.06.86 Bulletin 86/26**

(45) Mention de la délivrance du brevet: **20.04.88 Bulletin 88/16**

(84) Etats contractants désignés: **AT BE CH DE FR GB IT LI LU NL SE**

(56) Documents cités: **FR - A - 2 504 530**

(73) Titulaire: **UNIVERSITE LOUIS PASTEUR (STRASBOURG I), 4, rue Blaise Pascal, F-67070 Strasbourg Cédex (FR)**

(72) Inventeur: **ROBERT, Dominique, 248, avenue de la Vaugine, F-83300 Draguignan (FR)**
Inventeur: **JUNG, Louis, 205, route d'Oberhausbergen, F-67200 Strasbourg (FR)**

(74) Mandataire: **Ores, Irène et al, CABINET ORES 6, Avenue de Messine, F-75008 Paris (FR)**

EP 0 185 066 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne des dérivés soufrés de l'acide para-méthoxycinnamique ne pénétrant pas dans le torrent circulatoire. Elle concerne également l'obtention desdits dérivés soufrés. L'invention a encore pour objet l'utilisation cosmétologique, et la protection de la peau humaine contre les radiations solaires; elle concerne également les compositions dermo-pharmaceutiques et cosmétiques les contenant.

Les dérivés de l'acide para-méthoxycinnamique répondant à la formule I ci-après:

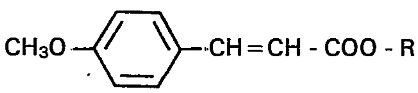 (I)

dans laquelle R représente des groupes alkyles non soufrés. Ces composés sont largement utilisés comme filtres solaires et contenus dans des compositions cosmétologiques à titre d'ingrédient actif.

Le para-méthoxycinnamate d'éthyle-2 hexyle est la molécule qui possède actuellement un des pouvoirs filtrants les plus sélectifs; elle est très largement utilisée dans les préparations antisolaires en France et dans le monde. Elle est reconnue par la FDA comme répondant aux critères nécessaires d'un filtre solaire sélectif.

Le Brevet n° 2 504 530, déposé le 28 Avril 1981 au nom de Pierre FABRE, a pour objet de nouveaux dérivés de l'acide para-méthoxycinnamique utiles en tant que filtres solaires et de formule I bis suivante:

$$CH_3O\text{-}C_6H_4\text{-}CH=CH\text{-}COO\text{-}(CH_2)_n\text{-}R \qquad \text{(I bis)}$$

Ces produits présentent cependant un inconvénient important dont les conséquences sont très désavantageuses: ils pénètrent dans le sang, comme le montre la thèse de Doctorat, mention Pharmacie, soutenue en 1982 par D. CLAUS à Strasbourg.

La présente invention concerne des dérivés soufrés de l'acide para-méthoxycinnamique présentant une activité de filtre solaire efficace et ne pénétrant pas dans le torrent circulatoire lorsqu'ils sont appliqués sur la peau. Les composés de l'invention répondent à la formule générale III ci-après:

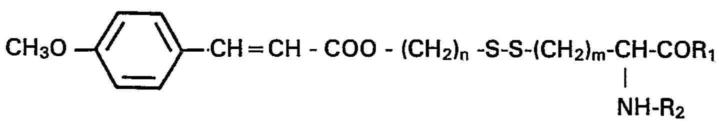 (III)

dans laquelle:

- la double liaison cinnamique peut exister soit sous la forme cis, soit sous la forme trans
- n est un nombre entier compris entre 1 et 6
- m est un nombre entier compris entre 1 et 6, et est de préférence égal à 1 ou 2
- $R_1$ est un radical -OR, R étant un atome d'hydrogène ou un groupe alkyle ou aryle, $R_1$ pouvant également être un amino-alkyle ou amino-aryle, ou présenter une structure amino-acide donnant une liaison peptidique
- $R_2$ représente un atome d'hydrogène ou un groupe alkyle ou aryle ou un groupe de formule R'-CO-, R' étant un groupe alkyle ou aryle ou encore R'-CO- présentant une structure acide aminé donnant une liaison peptidique.

Dans la présente description, le terme «alkyle» désigne des groupes hydrocarbonés aliphatiques contenant 1 à 12 atomes de carbone, à chaîne droite ou ramifiée. On préfère les groupes alkyle inférieur, c'est-à-dire les groupes alkyle contenant 1 à 4 atomes de carbone.

Le terme «aryle» désigne les groupes aromatiques non hétérocycliques du type phényle, benzyle et les homologues supérieurs, substitués ou non, ainsi que les groupes aromatiques hétérocycliques ayant 4 à 7 atomes de carbone dans le cycle aromatique et 1 à 4 hétéroatomes pouvant être l'oxygène, l'azote, le soufre, du type furane, pyridine oxazole, ainsi que leurs dérivés saturés, respectivement cyclohexane et tétrahydrofuranne, pipéridine, oxazolidine.

La présente invention à également pour objet un procédé de synthèse des dérivés soufrés de l'acide para-méthoxycinnamique à pont disulfure et à groupement aminoacide, de formule III ci-dessus, qui est caractérisé en ce qu'il consiste à faire réagir un composé pris dans le groupe qui comprend les acides aminés soufrés et les peptides présentant un groupement SH libre, ainsi que leurs dérivés, avec du para-méthoxycinnamate de thio-2-éthanol de formule II ci-après:

$$CH_3O\text{-}C_6H_4\text{-}CH=CH\text{-}COO\text{-}CH_2\text{-}CH_2\text{-}SH \qquad \text{(II)}$$

Selon un mode de mise en œuvre avantageux du procédé conforme à l'invention, le para-méthoxycinnamate de thio-2-éthanol, de formule II, utilisé pour préparer les composés de formule III conformes à la présente invention est lui-même obtenu en faisant réagir, au cours d'une première étape, du chlorure de thionyle sur de l'acide para-méthoxycinnamique, pour obtenir le chlorure de l'acide para-méthoxycinnamique que l'on fait réagir, au cours d'une deuxième étape, avec du dithio-2,2'-diéthanol pour obtenir le bis-(para-méthoxycinnamate) de dithio-2,2'-diéthanol qui répond à la formule IV ci-après:

$$CH_3O\text{-}C_6H_4\text{-}CH=CH\text{-}COO\text{-}CH_2\text{-}CH_2\text{-}S\text{-}S\text{-}CH_2\text{-}CH_2\text{-}OCO\text{-}CH=CH\text{-}C_6H_4\text{-}OCH_3 \qquad \text{(IV)}$$

qui est réduit, au cours d'une troisième étape, en para-méthoxycinnamate de thio-2-éthanol der formule II.

Le para-méthoxycinnamate de thio-2-éthanol de formule II est utilisé en qualité d'intermédiaire pour la préparation des composé de formule III.

Selon un mode de mise en œuvre du procédé de préparation des composés de formule III conforme à la présente invention, l'acide aminé soufré utilisé est la cystéine ou l'un de ses homologues supérieurs tel que l'homocystéine, ou un dérivé de ces acides aminés.

Selon un autre mode de mise en œuvre du procédé de préparation des composés de formule III conforme à la présente invention, la réaction de l'acide aminé ou du protide soufré avec le para-méthoxycinnamate de thio-2-éthanol de formule II est réalisée en présence d'iode dans un milieu aqueux approprié.

Selon un mode de mise en œuvre du procédé de préparation du para-méthoxycinnamate de thio-2-éthanol de formule II utilisé en tant qu'intermédiaire pour la préparation des composés de formule III, la réduction du bis-(para-méthoxycinnamate) de dithio-2,2'-diéthanol de formule IV en para-méthoxycinnamate de thio-2-éthanol de formule II est réalisée à l'aide de borohydrure de sodium, dans un milieu aqueux approprié.

Les composés de formule III conformes à la présente invention possèdent des propriétés de filtres solaires intéressants. Ils présentent un maximum d'efficacité tant au niveau de leur capacité d'absorption ultra-violette qu'à celui de leur fixation sur la peau. Leur spectre d'absorption se situe dans la zone des U.V.B. érythématogènes avec un maximum vers 310 nm; ils laissent passer les rayons U.V.A.

Il s'est, de même, avéré que les composés de formule II utilisés comme intermédiaires pour la préparation des composés de formule III présentent également des propriétés de filtres solaires.

Une étude in vivo de la fixation des filtres soufrés de formules III et II à la couche cornée a été réalisée sur la peau de lapin. Aucune trace d'acide para-méthoxycinnamique, métabolite de tout filtre solaire de formules III ou II, n'a pu être décelée dans le sang des lapins traités. La méthode d'analyse par chromatographie en phase gazeuse couplée à la spectrométrie de masse permet de déceler des concentrations de l'ordre de 10 pg. L'on peut donc en conclure que ces composés ne pénètrent pas dans le torrent circulatoire, contrairement au para-méthoxycinnamate d'éthyle-2-hexyle évoqué dans le préambule ainsi que les produits obtenus à partir du Brevet Pierre FABRE.

L'invention va maintenant être décrite plus en détail dans les Exemples qui vont suivre, qui n'ont aucun caractère limitatif.

*Exemple I*

A. *Synthèse du bis-(para-méthoxycinnamate) de dithio-2,2' diéthanol de formule IV*

– première étape: synthèse du chlorure d'acide para-méthoxycinnamique en présence de chlorure de thionyle.

Mode opératoire:

Dans un ballon muni d'un tube réfrigérant, 20 g d'acide para-méthoxycinnamique, 30 g de chlorure de thionyle et 200 ml de benzène sont portés à ébullition. Après 8 h de reflux, le solvant est évaporé à l'aide d'un évaporateur rotatif. Le résidu de chlorure de thionyle est éliminé de la même manière par évaporations et reprises successives par du benzène.

– deuxième étape: réaction du chlorure d'acide avec du dithio-2,2'-diéthanol.

Mode opératoire:

Dans un ballon muni d'un tube réfrigérant, 20 g de chlorure d'acide para-méthoxycinnamique, 8 g de dithio-2,2'-diéthanol et 200 ml de benzène sont portés à ébullition, Après 1 h de reflux, le solvant est évaporé à l'aide d'un évaporateur rotatif. L'ester obtenu est purifié par deux recristallisations successives dans de l'éthanol à 100°C.

– troisième étape: synthèse du para-méthoxycinnamate de thio-2-éthanol de formule II.

Dans un erlenmeyer, 1 g de composé III est dissous dans un mélange eau-acétonitrile (10 : 50); 1 g de $NaBH_4$ est ajouté par fractions. Après 30 minutes d'agitation, 50 ml d'une solution 0,1 molaire de $NH_4Cl$ sont ajoutés. Après filtration, le produit obtenu est séché. Le rendement en composé IV est de l'ordre de 50%.

Propriétés du composé de formule II

- poudre de Point de fusion de 98°C
- le maximum d'absorption U.V. se situe à 310 nm
- spectre IR ($CHCl_3$): 1635 $cm^{-1}$ (c=c); 1660 $m^{-1}$ ($C_6H_4$); 1700 $cm^{-1}$ (-co-o-)
- spectre RMN ($CDCl_3$): en ppm 1,55 (t, 2H, $CH_2$-S); 2,45 (t, 1H-SH); 3,72 (S, 3H, $OCH_3$); 4,40 (t, 2H, -$CH_2$-CO-); 6,25 (d, 1H, -CH-CO) et 7,65 (d, 1H, -CH-Ar): couplage 16 Hz (dérivé trans); 6,82 (d, 2H) et 7,45 (d, 2H): couplage AA'BB'
- constantes chromatographiques : sur couche mince de gel de Silice Merck 60F254 Rf = 0,95 solvant de migration: chloroforme - acétate d'éthyle (50 : 50).

B. *Synthèse du composé de formule III, $n = 2$, $m = 1$, $R_2 = H$ et $R_1 = OH$*

Dans un erlenmeyer, 2,7 g de composé IV sont agités avec 2,3 g de cystéine en présence de 50 ml d'acétonitrile et 10 ml d'eau distillée. 2,3 g diode pulvérisés sont ajoutés progressivement toujours sous agitation qui est maintenue entre 2 et 4 heures. La séparation de composé II ($n = 2$, $m = 1$, $R_2 = H$ et $R_1 = OH$) se fait sur colonne de silice le solvant d'élution étant par exemple un mélange de chloroforme-méthanol-benzène (10 : 5 : 20).

Propriétés du composé de formule IV ($n = 2$, $m = 1$, $R_2 = H$ et $R_1 = OH$)

- poudre jaunâtre à point de fusion difficile à déterminer vu la décomposition
- le maximum d'absorption U.V. se situe à 310 nm
- avec le réactif à base de ninhydrine on obtient la réaction caractéristique des acides aminés
- les spectre IR et RMN donnent les bandes caractéristiques du groupement para-méthoxycinnamique déjà décrit pour le composé IV
- constantes chromatographiques: sur couche mince de gel de silice Merck 60F254 Rf: 0,35,

solvant de migration: chloroforme-méthanol-benzène (10:5:20).

*Exemple II*

*Compositon dermo-pharmaceutique contenant un composé présentant des propriétés de filtre solaire conforme à la présente invention*

Le composé présentant des propriétés de filtre solaire, conforme à la présente invention est incorporé à la concentration 5% dans une émulsion qui peut avoir par exemple la composition suivante:

| | | |
|---|---|---|
| Paraffine | 12 | g |
| Mono- et distéarate de PEG (Téfose 1500) | 7 | g |
| Acide stéarique | 1 | g |
| Alcool cétylique | 0,5 | g |
| Glycéride parmitostéarique polyoxyéthyléné (Labrafil M 2130) | 3 | g |
| Eau q.s.p. | 100 | g |

*Méthode de recherche de l'acide para-méthoxycinnamique (métabolite) dans le sang de lapin après application des filtres solaires soufrés conformes à l'invention incorporé dans l'émulsion précédente.*

– Conditions d'application et préjèvement plasmatiques

2 g d'émulsion à tester sont appliqués sur la peau épilée du lapin. Après uniformisation du dépôt par massage, des prélèvements sanguins de 6 ml sont effectués à différents temps après le dépôt. Les mesures sont effectuées sur la fraction plasmatique.

– Déterminations quantitatives en CPG(SM

Le filtre solaire présentant une fonction ester qui est hydrolysée par les estérases sanguines, on a recherché dans les fractions plasmatiques la présence du métabolite qu'est l'acide para-méthoxycinnamique.

On a utilisé comme étalon interne, l'acide cinnamique qui est ajouté au plasma. Ce dernier est acidifié puis extrait par un mélange acétone-chlorure de méthylène (1;3), les extraits sont ensuite injectés après dérivatisation avec du BSTFA.

Dans tous les extraits étudiés, la recherche d'acide para-méthoxycinnamique s'est révélée négative. La sensibilité de la méthode permet de détecter jusqu'à 2 pg par injection.

Conclusion:

Le filtre soufré ne passe plus dans le sang alors que le PMCEH appliqué dans les mêmes conditions y est retrouvé sous forme d'acide para-méthoxycinnamique à une concentration comprise entre le ng et plusieurs µg.

**Revendications pour les Etats contractants:**
BE, CH, DE, FR, GB, IT, LI, LU, NL, SE

1. Dérivés soufrés de l'acide para-méthoxycinnamique, caractérisés en ce qu'ils répondent à la formule générale III ci-après:

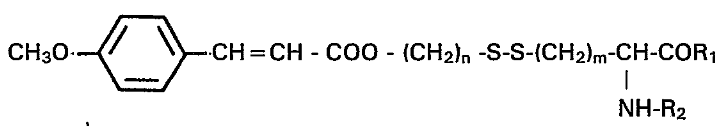

(III)

dans laquelle:
- la double liaison cinnamique peut exister soit sous la forme cis, soit sous la forme trans
- n est un nombre entier compris entre 1 et 6
- m est un nombre entier compris entre 1 et 6, et est de préférence égal à 1 ou 2
- $R_1$ est un radical OR, R étant un atome d'hydrogène ou un groupe alkyle ou aryle, $R_1$ pouvant également être un amino-alkyle ou amino-aryle, ou présenter une structure amino-acide donnant une liaison peptidique
- $R_2$ représente un atome d'hydrogène ou un groupe alkyle ou aryle ou un groupe de formule R'-CO-, R' étant un groupe alkyle ou aryle ou encore R'-CO- présentant une structure acide aminé donnant une liaison peptidique.

2. Dérivé soufré de l'acide para-méthoxycinnamique selon la revendication 1, caractérisé en ce qu'il s'agit de l'acide amino-2 [(p-méthoxycinnamoyloxy-2 éthyl)-2 disulfinyl]-3-propionique de formule III ci-dessus.

3. Procédé pour l'obtention de dérivés soufrés de l'acide para-méthoxycinnamique à pont disulfure et à groupement amino-acide, selon les revendications 1 à 2, caractérisé en ce qu'il consiste à faire réagir un composé pris dans le groupe qui comprend les acides aminés soufrés et les peptides présentant un groupement SH libre, ainsi que leurs dérivés, avec du para-méthoxycinnamate de thio-2-éthanol de formule II ci-après:

$$CH_3O\text{-}C_6H_4\text{-}CH=CH\text{-}COO\text{-}CH_2\text{-}CH_2\text{-}SH \qquad (II)$$

4. Procédé selon la revendication 3, caractérisé en ce que le para-méthoxycinnamate de thio-2-éthanol de formule II mise en œuvre pour l'obtention des dérivés de formule III selon l'une quelconque des revendications 1 ou 2, est obtenu au cours d'étapes préalables en faisant réagir du chlorure de thionyle sur de l'acide para-méthoxycinnamique que l'on fait ensuite réagir avec du dithio-2,2'-diéthanol pour obtenir le bis-(para-méthoxycinnamate) de dithio-2,2'-diéthanol qui répond à la formule IV ci-après:

$$CH_3O\text{-}C_6H_4\text{-}CH=CH\text{-}COO\text{-}CH_2\text{-}CH_2\text{-}S\text{-}S\text{-}CH_2\text{-}CH_2\text{-}OCO\text{-}CH=CH\text{-}C_6H_4\text{-}OCH_3 \qquad (IV)$$

et à partir duquel on obtient par réduction le para-méthoxycinnamate de thio-2-éthanol de formule II.

5. Procédé selon la revendication 3, caractérisé en ce que l'acide aminé soufré utilisé est la cystéine ou l'un de ses homologues supérieurs tel que l'homocystéine, ou un dérivé de ces amides aminés.

6. Procédé selon la revendication 3 ou la revendication 5, caractérisé en ce que la réaction de l'acide aminé ou du protide soufré avec le para-méthoxycinnamate de thio-2-éthanol de formule II est réalisée en présence d'iode dans un milieu aqueux approprié.

7. Procédé selon la revendication 4, caractérisé en ce que la réduction du bis-(para-méthoxycinnamate) de dithio-2,2'-diéthanol de formule IV en paraméthoxycinnamate de thio-2-éthanol de formule II est réalisée à l'aide de boro-hydrure de sodium, dans un milieu aqueux approprié.

8. Compositions dermopharmaceutiques ou cosmétologiques caractérisées en ce qu'elles contiennent, à titre d'ingrédient actif présentant des proriétés de filtre solaire à activité topique ne pénétrant pratiquement pas dans le torrent circulatoire, un composé de formule générale III selon l'une quelconque des revendications 1 ou 2, en combinaison avec un véhicule approprié pour l'application sur la surface de la peau de l'homme.

**Revendications pour l'Etat contractant:** AT

1. Procédé pour l'obtention de dérivés soufrés de l'acide para-méthoxycinnamique répondant à la formule générale III ci-après:

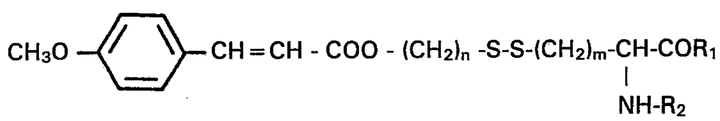

(III)

dans laquelle:
- la double liaison cinnamique peut exister soit sous la forme cis, soit sous la forme trans
- n est un nombre entier compris entre 1 et 6
- m est un nombre entier compris entre 1 et 6, et est de préférence égal à 1 ou 2
- $R_1$ est un radical OR, R étant un atome d'hydrogène ou un groupe alkyle ou aryle, $R_1$ pouvant également être un amino-alkyle ou amino-aryle, ou présenter une structure amino-acide donnant une liaison peptidique
- $R_2$ représente un atome d'hydrogène ou un groupe alkyle ou aryle ou un groupe de formule R'-CO-, R' étant un groupe alkyle ou aryle ou encore R'-CO- présentant une structure acide aminé donnant une liaison peptidique,

caractérisé en ce qu'il consiste à faire réagir un composé pris dans le groupe qui comprend les acides aminés soufrés et les peptides présentant un groupement SH libre, ainsi que leurs dérivés, avec du paraméthoxycinnamate de thio-2-éthanol de formule II ci-après:

$$CH_3O\text{-}C_6H_4\text{-}CH=CH\text{-}COO\text{-}CH_2\text{-}CH_2\text{-}SH \qquad (II)$$

2. Procédé pour l'obtentionde dérivés soufrés selon la revendication 1, caractérisé en ce qu'il s'agit de l'acide amino-2 [(p-méthoxycinnamoyloxy-2 éthyl)-2 disulfinyl]-3-propionique de formule III ci-dessus.

3. Procédé selon la revendication 1, caractérisé en ce que le para-méthoxycinnamate de thio-2-éthanol de formule II mis en œuvre pour l'obtention des dérivés de formule III selon la revendication 1, est obtenu au cours d'étapes préalables en faisant réagir du chlorure de thionyle sur de l'acide para-méthoxycinnamique, pour obtenir le chlorure de l'acide paraméthoxycinnamique que l'on fait ensuite réagir avec du dithio-2,2'-diéthanol pour obtenir le bis-(paraméthoxycinnamate) de dithio-2,2'-diéthanol qui répond à la formule IV ci-après:

$$CH_3O\text{-}C_6H_4\text{-}CH=CH\text{-}COO\text{-}CH_2\text{-}CH_2\text{-}S\text{-}S\text{-}CH_2\text{-}CH_2\text{-}OCO\text{-}CH=CH\text{-}C_6H_4\text{-}OCH_3 \qquad (IV)$$

et à partir duquel on obtient par réduction le paraméthoxycinnamate de thio-2-éthanol de formule II.

4. Procédé selon la revendication 1, caractérisé en ce que l'acide aminé soufré utilisé est la cystéine ou l'un de ses homologues supérieurs, tels que l'homocystéine. ou un dérivé de ces acides aminés.

5. Procédé selon la revendication 1, caractérisé en ce que la réaction de l'acide aminé ou du protide soufré avec le para-méthoxycinnamate de thio-2-éthanol de formule II est réalisée en présence d'iode dans un milieu aqueux approprié.

6. Procédé selon la revendication 3, caractérisé en ce que la réduction du bis-(para-méthoxycinnamate) de dithio-2,2'-diéthanol de formule IV en paraméthoxycinnamate de thio-2-éthanol de formule II est réalisée à l'aide de borohydrure de sodium, dans un milieu aqueux approprié.

**Patentansprüche für die Vertragsstaaten:**
BE, CH, DE, FR, GB, IT, LI, LU, NL, SE

1. Schwefel enthaltende Derivate von p-Methoxyzimtsäure, dadurch gekennzeichnet, dass sie der folgenden allgemeinen Formel III entsprechen:

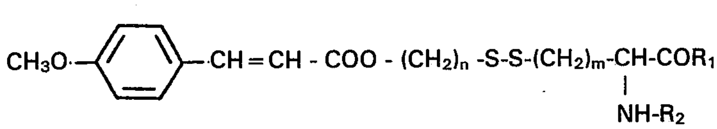

(III)

in der
- die Zimtsäuredoppelbindung sowohl in cis- als auch in trans-Stellung vorliegen kann,
- n eine ganze Zahl von 1 bis 6 bedeutet,
- m eine ganze Zahl von 1 bis 6 und vorzugsweise gleich 1 oder 2 ist,
- $R_1$ für einen Rest OR steht, wobei R ein Wasserstoffatom oder eine Alkyl- oder Arylgruppe ist, $R_1$ gleichermassen eine Aminoalkyl- oder Aminoarylgruppe bedeuten kann oder eine Aminosäurestruktur, welche eine Peptidbindung ergibt, darstellen kann,
- $R_2$ für ein Wasserstoffatom oder eine Alkyl- oder Arylgruppe oder eine Gruppe der Formel R'-CO- steht, in der R' eine Alkyl- oder Arylgruppe bedeutet, oder R'-CO- auch eine Aminosäurestruktur, welche eine Peptidbindung ergibt, darstellen kann.

2. Schwefel enthaltendes Derivat von p-Methoxyzimtsäure nach Anspruch 1, dadurch gekennzeichnet, dass es sich um Amino-2-[(p-methoxycinnamoyloxy-2-ethyl)-2-disulfinyl]-3-propionsäure der obigen Formel III handelt.

3. Verfahren zur Herstellung von Schwefel enthaltenden Derivaten von p-Methoxyzimtsäure mit Dischwefelbrücke und Aminosäuregruppierung nach Anspruch 1 oder 2, dadurch gekennzeichnet, dass das Verfahren darin besteht, dass man eine Zusammensetzung, ausgewählt aus der Gruppe, umfassend Schwefel enthaltende Aminosäuren und Peptide, die eine freie SH-Gruppierung aufweisen, sowie deren Derivate, mit p-Methoxycinnamat von Thio-2-ethanol der folgenden Formel II:

$$CH_3O\text{-}C_6H_4\text{-}CH=CH\text{-}COO\text{-}CH_2\text{-}CH_2\text{-}SH \qquad (II)$$

umsetzt.

4. Verfahren nach Anspruch 3, dadurch gekennzeichnet, dass das p-Methoxycinnamat von Thio-2-ethanol der Formel II, welches zum Erhalt der Derivate der Formel III nach einem der Ansprüche 1 oder 2 verwendet wird, im Verlauf vorhergehender Stufen erhalten worden ist, wobei Thionylchlorid mit p-Methoxyzimtsäure umgesetzt wird, um p-Methoxyzimtsäurechlorid zu erhalten, welches man schliesslich mit Dithio-2,2'-diethanol umsetzt, wodurch das Bis(p-methoxycinnamat) von Dithio-2,2'-diethanol, das der folgenden Formel IV entspricht:

$$CH_3O\text{-}C_6H_4\text{-}CH=CH\text{-}COO\text{-}CH_2\text{-}CH_2\text{-}S\text{-}S\text{-}CH_2\text{-}CH_2\text{-}OCO\text{-}CH=CH\text{-}C_6H_4\text{-}OCH_3 \qquad (IV)$$

erhalten wird, aus dem man durch Reduktion das p-Methoxycinnamat von Thio-2-ethanol der Formel II erhält.

5. Verfahren nach Anspruch 3, dadurch gekennzeichnet, dass die verwendete Schwefel enthaltende Aminosäure das Cystein oder eines seiner höheren Homologen, wie das Homocystein, oder ein Derivat dieser Aminsäuren ist.

6. Verfahren nach Anspruch 3 oder 5, dadurch gekennzeichnet, dass die Umsetzung der Schwefel enthaltenden Aminosäure oder Protids mit dem p-Methoxycinnamat von Thio-2-ethanol der Formel II in Gegenwart von Jod in einem geeigneten wässrigen Medium durchgeführt wird.

7. Verfahren nach Anspruch 4, dadurch gekennzeichnet, dass die Reduktion des Bis-(p-methoxycinnamats) von Dithio-2,2'-diethanol der Formel IV zu p-Methoxycinnamat von Thio-2-ethanol der Formel II mit Hilfe von Natriumborhydrid in einem geeigneten wässrigen Medium durchgeführt wird.

8. Dermopharmazeutische oder kosmetologische Zusammensetzungen, dadurch gekennzeichnet, dass sie als aktiven Bestandteil, der Sonnenlichtfiltereigenschaften dahingehend hat, dass die topische Aktivität praktisch nicht in den Blutkreislauf eindringt, eine Zusammensetzung der allgemeinen Formel III nach einem der Ansprüche 1 oder 2 in Verbindung mit einem geeigneten Träger zur Aufbringung auf die menschliche Hautoberfläche enthalten.

**Patentansprüche für den Vertragsstaat:** AT

1. Verfahren zur Herstellung von Schwefel enthaltenden Derivaten von P-Methoxyzimtsäure der folgenden allgemeinen Formel III:

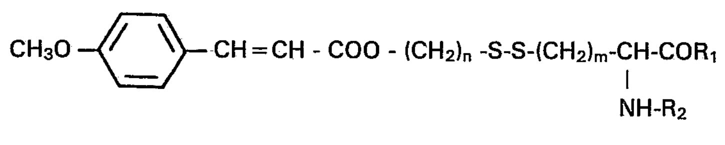

(III)

in der
- die Zimtsäuredoppelbindung sowohl in cis- als auch in trans-Stellung vorliegen kann,
- n eine ganze Zahl von 1 bis 6 bedeutet,
- m eine ganze Zahl von 1 bis 6 und vorzugsweise gleich 1 oder 2 ist,
- $R_1$ für einen Rest OR steht, wobei R ein Wasserstoffatom oder eine Alkyl- oder Arylgruppe ist, $R_1$ gleichermassen eine Aminoalkyl- oder Aminoarylgruppe bedeuten kann oder eine Aminosäurestruktur, welche eine Peptidbindung ergibt, darstellen kann,
- $R_2$ für ein Wasserstoffatom oder eine Alkyl- oder Arylgruppe oder eine Gruppe der Formel R'-CO- steht, in der R' eine Alkyl- oder Arylgruppe bedeutet, oder R'-CO-auch eine Aminosäurestruktur, welche eine Peptidbindung ergibt, darstellen kann,

dadurch gekennzeichnet, dass das Verfahren darin besteht, dass man eine Zusammensetzung, ausgewählt aus der Gruppe, umfassend Schwefel enthaltende Aminosäuren und Peptide, die eine freie SH-Gruppierung aufweisen, sowie deren Derivate, mit p-Methoxycinnamat von Thio-2-ethanol der folgenden Formel II:

$$CH_3O\text{-}C_6H_4\text{-}CH=CH\text{-}COO\text{-}CH_2\text{-}CH_2\text{-}SH \qquad (II)$$

umsetzt.

2. Verfahren zur Herstellung von Schwefel enthaltenden Derivaten nach Anspruch 1, dadurch gekennzeichnet, dass es sich um Amino-2-[(p-methoxycinnamoyloxy-2-ethyl)-2-disulfinyl]-3-propionsäure der obigen Formel III handelt.

3. Verfahren nach Anspruch 1, dadurch gekennzeichnet, dass das p-Methoxycinnamat von Thio-2-ethanol der Formel II, welches zur Herstellung der Derivate der Formel III nach Anspruch 1 eingesetzt wird, im Verlauf vorhergehender Stufen erhalten worden ist, wobei Thionylchlorid mit p-Methoxyzimtsäure umgesetzt wird, um p-Methoxyzimtsäurechlorid zu erhalten, welches man schliesslich mit Dithio-2,2'-diethanol umsetzt, wodurch das Bis(p-methoxycinnamat) von Dithio-2,2'-diethanol, das der folgenden Formel IV entspricht:

$$CH_3O\text{-}C_6H_4\text{-}CH=CH\text{-}COO\text{-}CH_2\text{-}CH_2\text{-}S\text{-}S\text{-}CH_2\text{-}CH_2\text{-}OCO\text{-}CH=CH\text{-}C_6H_4\text{-}OCH_3 \quad \text{(IV)}$$

erhalten wird, aus dem man durch Reduktion das p-Methoxycinnamat von Thio-2-ethanol der Formel II erhält.

4. Verfahren nach Anspruch 1, dadurch gekennzeichnet, dass die verwendete Schwefel enthaltende Aminosäure das Cystein oder eines seiner höheren Homologen, wie das Homocystein, oder ein Derivat dieser Aminsäuren ist.

5. Verfahren nach Anspruch 1, dadurch gekennzeichnet, dass die Umsetzung der Schwefel enthaltenden Aminosäure oder des Schwefel enthaltenden Protids mit dem p-Methoxycinnamat von Thio-2-ethanol der Formel II in Gegenwart von Jod in einem geeigneten wässrigen Medium durchgeführt wird.

6. Verfahren nach Anspruch 3, dadurch gekennzeichnet, dass die Reduktion des Bis-(p-methoxycinnamats) von Dithio-2,2'-diethanol der Formel IV zu p-Methoxycinnamat von Thio-2-ethanol der Formel II mit Hilfe von Natriumborhydrid in einem geeigneten wässrigen Medium durchgeführt wird.

**Claims for the Contracting States:**
BE, CH, DE, FR, GB, IT, LI, LU, NL, SE

1. Sulphur-containing derivatives of paramethoxycinnamic acid, characterized in that they correspond to the general formula III below:

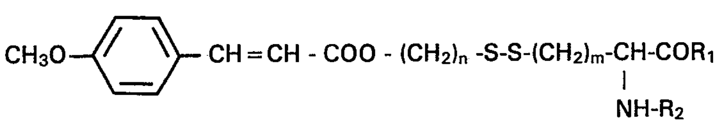

(III)

in which:
- the cinnamic duble bond can exist either in the cis form or in the trans form,
- n is an integer between 1 and 6,
- m is an integer between 1 and 6 and is preferably equal to 1 or 2,
- $R_1$ is a radical OR, in which R is a hydrogen atom or an alkyl or aryl group, it also being possible for $R_1$ to be an aminoalkyl or aminoaryl group or to have an amino acid structure giving a peptide bond, and
- $R_2$ represents a hydrogen atom, an alkyl or aryl group or a group of the formula R'-CO-, R' being an alkyl or aryl group or R'-CO- having an amino acid structure giving a peptide bond.

2. A sulphur-containing derivative of paramethoxycinnamic acid according to claim 1, characterized in that it is 2-amino-3-[2-(2-p-methoxycinnamoyloxyethyl)-disulphinyl]propionic acid of the formula III above.

3. A process for the preparation of sulphur-containing derivatives of paramethoxycinnamic acid having a disulphide bridge and an amino acid group, according to claims 1 and 2, characterized in that it consists in reacting a compound selected from the group comprising sulphur-containing amino acids and peptides having a free SH group, as well as derivatives thereof, with 2-thioethyl paramethoxycinnamate of the formula II below:

$$CH_3O\text{-}C_6H_4\text{-}CH=CH\text{-}COO\text{-}CH_2\text{-}CH_2\text{-}SH \quad \text{(II)}$$

4. The process according to claim 3, characterized in that the 2-thioethyl paramethoxycinnamate of the formula II used to obtain the derivatives of the formula III according to either one of claims 1 or 2 is obtained in previous steps by reacting thionyl chloride with paramethoxycinnamat acid to give paramethoxycinnamoyl chloride, which is then reacted with 2,2'-dithiodiethanol to give 2,2'-dithiodiethyl bis(paramethoxycinnamate) corresponding to the formula IV below:

$$CH_3O\text{-}C_6H_4\text{-}CH=CH\text{-}COO\text{-}CH_2\text{-}CH_2\text{-}S\text{-}S\text{-}CH_2\text{-}CH_2\text{-}OCO\text{-}CH=CH\text{-}C_6H_4\text{-}OCH_3 \quad \text{(IV)}$$

from which 2-thioethyl paramethoxycinnamate of the formula II is obtained by reduction.

5. The process according to claim 3, characterized in that the sulphur-containing amino acid used is cysteine or one of its higher homologues such as homocysteine, or a derivative of these amino acids.

6. The process according to claim 3 or claim 5, characterized in that the reaction of the sulphur-containing amino acid or protide with the 2-thioethyl paramethoxycinnamate of the formula II is performed in the presence of iodine in a suitable aqueous medium.

7. The process according to claim 4, characterized in that the 2,2'-dithiodiethyl bis(paramethoxycinnamate) of the formula IV is reduced to the

2-thioethyl paramethoxycinnamate of the formula II with sodium borohydride in a suitable aqueous medium.

8. Dermopharmaceutical or cosmetological compositions, characterized in that they contain, as an active ingredient possessing sunscreen properties with a topical action, which practically does not enter the bloodstream, a compound of the general formula III according to either one of claims 1 or 2, in combination with a vehicle suitable for application to the surface of human skin.

**Claims for the Contracting State:** AT

1. A process for the preparation of sulphur-containing derivatives of paramethoxycinnamic acid, corresponding to the general formula III below:

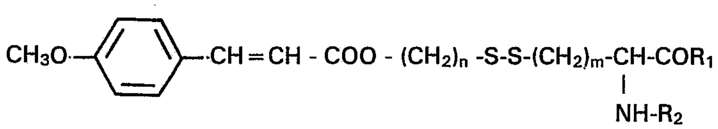

(III)

in which:

- the cinnamic duble bond can exist either in the cis form or in the trans form,
- n is an integer between 1 and 6,
- m is an integer between 1 and 6 and is preferably equal to 1 or 2,
- $R_1$ is a radical OR, in which R is a hydrogen atom or an alkyl or aryl group, it also being possible for $R_1$ to be an aminoalkyl or aminoaryl group or to have an amino acid structure giving a peptide bond, and
- $R_2$ represents a hydrogen atom, an alkyl or aryl group or a group of the formula R'-CO-, R' being an alkyl or aryl group or R'-CO- having an amino acid structure giving a peptide bond,

characterized in that it consists in reacting a compound selected from the group comprising sulphur-containing amino acids and peptides having a free SH group, as well as derivatives thereof, with 2-thioethyl paramethoxycinnamate of the formula II below:

$$CH_3O\text{-}C_6H_4\text{-}CH=CH\text{-}COO\text{-}CH_2\text{-}CH_2\text{-}SH \qquad (II)$$

2. The process for the preparation of sulphur-containing derivatives according to claim 1, characterized in that the sulphur-containing derivative is 2-amino-3-[2-(2-p-methoxycinnamoyloxyethyl)-disulphinyl]-propionic acid of the formula III above.

3. The process according to claim 1, characterized in that the 2-thioethyl paramethoxycinnamate of the formula II used to obtain the derivatives of the formula III according to claim 1 is obtained in previous steps by reacting thionyl chloride with paraethoxycinnamic acid to give paramethoxycinnamoyl chloride, which is then reacted with 2,2'-dithiodiethanol to give 2,2'-dithiodiethyl bis(paramethoxycinnamate) corresponding to the formula IV below:

$$CH_3O\text{-}C_6H_4\text{-}CH=CH\text{-}COO\text{-}CH_2\text{-}CH_2\text{-}S\text{-}S\text{-}CH_2\text{-}CH_2\text{-}OCO\text{-}CH=CH\text{-}C_6H_4\text{-}OCH_3 \qquad (IV)$$

from which 2-thioethyl paramethoxycinnamate of the formula II is obtained by reduction.

4 The process according to claim 1, characterized in that the sulphur-containing amino acid used is cysteine or one of its higher homologues such as homocysteine, or a derivative of these amino acids.

5. The process according to claim 1, characterized in that the reaction of the sulphur-containing amino acid or protide with the 2-thioethyl paramethoxycinnamate of the formula II is performed in the presence of iodine in a suitable aqueous medium.

6. The process according to claim 5, characterized in that the 2,2'-dithiodiethyl bis(paramethoxycinnamate) of the formula IV is reduced to the 2-thioethyl paramethoxycinnamate of the formula II with sodium borohydride in a suitable aqueous medium.